# EUROPEAN PATENT APPLICATION

(11) **EP 1 598 090 A1**
(43) Date of publication of application: **23.11.2005**
(21) Application number: 04707658.3
(22) Date of filing: 03.02.2004
(51) Int. Cl.: A61M 25/10

(54) **BALLOON FOR INTRAAORTIC BALLOON PUMPING CATHETER, CATHETER FITTED WITH THE SAME AND PROCESS FOR PRODUCING THE BALLOON**

(30) Priority: 14.02.2003 JP 2003036076
(71) Applicant: ZEON CORPORATION, Tokyo 100-8323 (JP)
(72) Inventor: SAKAI, Koichi, c/o Zeon Medical Inc., Tokyo 1050011 (JP)
(74) Representative: Banzer, Hans-Jörg
(86) International application number: PCT/JP2004/001026
(87) International publication number: WO 2004/071567

(57) **Abstract**

A balloon for use in the method of intra aortic balloon pumping (IABP), produced by blow molding of a polyether polyurethane whose 100% modulus is 5 to 18 MPa, the balloon having a film thickness of 30 to 80 µm and a 50% modulus in the longitudinal direction is 30 to 140 MPa.

## Description

### TECHNICAL FIELD

The present invention relates to a balloon for catheter used for intra aortic balloon pumping (hereinafter referred to as "IABP") method, catheter fitted with the same, and process for producing the same.

### BACKGROUND OF THE INVENTION

### [Prior Art]

IABP is a treatment method to assist cardiac function when the cardiac function decline due to cardiac insufficiency etc. by inserting a balloon catheter in the aorta, synchronizing the balloon placed at the end of catheter with the beating of the heart, i.e. deflating the balloon in systole and inflating the same when in diastole, and increasing coronary blood flow. This IABP treatment method may take more than one month. Therefore, the method requires high blood compatibility.

Further, calcification may occur at inner side of blood vessel in patients treated by IABP method due to decline of the cardiac function. Therefore, during the inflation and deflation of the balloon along with the heartbeat as mentioned above, the balloon may contact with the calcification. When the balloon contacts the calcification, the balloon may be deteriorated and gas to inflate and deflate the balloon may be leaked to cause embolus. Accordingly, the balloon of IABP catheter requires sufficient wearing resistance not to cause deterioration by the contact with the calcification.

On the other, when percutaneous insertion is done with IABP catheter by Seldinger technique, the balloon is folded to insert into a body cavity and when film thickness of balloon is thick, outer diameter of the folded balloon becomes large. As a result, perforation of blood vessels required for the insert becomes large and burden to patients increases. Therefore, thin-film is required for the IABP catheter balloon in order to carry out low profiling (obtaining smaller diameter of balloon when it is folded).

Some patients may have extremely curved intra aortic. Therefore, IABP catheter balloon is required for its flexibility in order to inflate and defalte along with the formation of blood vessels even in the bended intra aortic.

Conventionally, IABP catheter balloon has been manufactured by dipping molding polyurethane (See Reference 1: Japanese Unexamined Patent Application No.5-92041 etc.), which is known as having high blood compatibility. Reference 1 has found that there is a correlation between modulus values and wearing resistance of the balloon. And said reference 1 describes that a balloon having a superior wearing resistance can be obtained by producing a balloon having initial 100% modulus of at least a fixed value using dipping molding etc. However, since polyurethane is essentially inferior to mechanical strength, even with above-mentioned molding, the balloon used for IABP method was required to thicken its film thickness in order to obtain required wearing resistance. Consequently, efficient low profiling of the balloon was difficult.

For the balloon used for IABP method having both blood compatibility and sufficient strength, 2 layered structure (See Reference 2: Japanese Unexamined Patent Application No.4-144572 etc.) molded by blow molding is known. Said 2 layeredballoon has an inner layer comprising crystalline plastic with high strength such as polyethylene terephthalate (PET) or polyamide and an outer layer comprising elastic material having blood compatibility such as polyurethane. However, since said balloon had 2 layered structure, it was difficult to make the film thinner, sufficient low-profiling was not possible, and molding was difficult.

### DISCLOSURE OF THE INVENTION

An object of the present invention is to provide a balloon and a catheter fitted with the same that are possible to follow bended blood vessels, to carry out low profiling, and to provide sufficient wearing resistance when used for IABP method even with a thin and one layer film.

Present inventor, in order to solve the above-mentioned object, has been investigating materials and molding method of the IABP catheter balloon in detail. Consequently, the inventor has found that the object can be achieved by obtaining a balloon by blow molding a polyether polyurethane having specific characteristic. And this lead to completion of the invention.

According to the invention, a balloon for IABP catheter manufactured by blow molding polyether polyurethane whose 100% modulus is 5 to 18 MPa, wherein the balloon has a film thickness of 30 to 80 µm and a 50% modulus in the longitudinal direction of 30 to 140 MPa is provided.

Breaking strength of the polyether polyurethane is preferably at least 30MPa.

Shore A hardness of the polyether polyurethane is preferably 80 to 97.

Further, according to the present invention, an IABP catheter having a balloon is provided. The balloon is able to inflate and deflate along with introduction and derivation of a fluid inside the balloon. The balloon is produced by blow molding polyether polyurethane whose 100% modulus is 5 to 18 MPa wherein the balloon has a film thickness of 30 to 80 µm and a 50% modulus in the longitudinal direction of 30 to 140 MPa.

The present invention will be explained below in detail based on embodiments of the invention.

The balloon of the invention is obtained by molding polyether polyurethane. Here, polyether polyurethane is a thermoplastic polyurethane elastomer wherein units including urethane bond or urea bond of diisocyanate and chain extension agent is hard segment, and polyether polyol is soft segment. The polyether polyurethane forms microfacies separation structure by concentrations among hard segments and among soft segments. The polyether polyurethane is highly compatible to blood due to said structure.

Diisocyanate compounds comprising hard segments of polyether polyurethane are not limi ted but 4 , 4' - diphenyl methane diisocyanate (MDI), MDI hydrogenation or hexamethylene diisocyanate can be used. Polyether polyol compounds comprising soft segments of polyether polyurethane are not particularly limited when the polyether has hydroxyl group at the end of molecule, but polyoxy tetramethylene glycol (PTMG) or polyoxy propylene glycol (PPG) etc. can be used. For chain extension agent, short-chained diol such as 1,4-butanediol or ethylene glycol or diamine such as ethylene diamine is used.

Polyether polyurethane used for blow molding of the balloon of the invention is required to have 100% modulus of 5 to 18 MPa. When said 100% modulus is less than 5 MPa, 50% modulus in the longitudinal direction of obtained balloon decreases and wearing resistance of the balloon becomes insufficient. When said 100% modulus is more than 18 MPa, 50% modulus in the longitudinal direction of obtained balloon excessively increases and that flexibility of the balloon becomes insufficient. 100% modulus of the polyether polyurethane is preferably 7 to 16 MPa, more preferably 8 to 12 MPa.

Further, polyether polyurethane used for blow molding the balloon of the invention preferably has a breaking strength of at least 30 MPa. By using such polyether polyurethane, balloon superior in wearing resistance and hard to be damaged by a burs t can be obtained. Breaking strength of the polyether polyurethane is preferably at least 35 MPa, more preferably at least 40 MPa. Upper limit of the breaking strength is not particularly limited but normally at most 60MPa considering such as difficulty of manufacturing the balloon.

Furthermore, polyether polyurethane used for blow molding the balloon of the invention preferably has shore A hardness of 80 to 97. By using such polyether polyurethane, a balloon superior in both wearing resistance and following ability to follow along bended blood vessels can be obtained. Shore A hardness of the polyether polyurethane is preferably 85 to 95, more preferably 88 to 92.

In the present invention, 100% modulus, breaking strength and shore A hardness of the polyether polyurethane used for blow molding of the balloon of the invention are calculated values obtained by manufacturing experimental piece and experimenting the same in accordance with JIS K-7311.

polyether polyurethane used for blow molding of the invention can be synthesized by prepolymer process, one-shot process or any other processes. Here, to obtain desirable 100% modulus of the polyether polyurethane, a method changing the ratio of the hard segment and the soft segment etc. can be adopted. Further, the polyether polyurethane usable for the invention can also be obtained commercially by a trade name "Pelesen" (Dow Chemical made) etc.

Balloon of the invention can be obtained by blow molding above-mentioned polyether polyurethane. Concrete method of the blow molding is not limited but can be done such as by following.

First, a parison having outer diameter of 3 to 6 mm and thickness of 0 . 3 to 2 mm is manufactured by polyether polyurethane with melt extrusion or so. Next, while heating said parison, drawing process is done by adding tension in the longitudinal direction of the parison. This drawing process can be done before inserting the parison into a balloon mold or when inserting the same into the balloon mold. Said drawing process is with magnification of 1. 0 to 2 . 0, preferably 1 . 1 to 1 . 6 . Further, heating temerature of the parison during the drawing process is 60 to 100 C° and preferably 70 to 90 C° . Next, by inserting the parison into the balloon shaped mold, impressing pressure of 0.5 to 1.2 MPa, preferably 0.7 to 1.0 MPa inside the parison, and heating the balloon mold while adding tension in the longitudinal direction of the parison, said parison expands. When said expanding parison is sufficiently expanded, the parison contacts the balloon mold. Then, under said condition, the mold is impressed and set leading to the completion of the balloon molding. Heating temperature of the balloon mold during the molding process is 110 to 190C° , preferably 130 to 170C ° . After the completion of balloon molding, the mold is sufficiently cooled to room temperature while impressing the parison. After the completion of cooling, impression to the parison is relieved and said molded balloon is removed.

Accordingly, when molding the balloon by said blow molding method, polyether polyurethane chain orients. Therefore, even composed with one layer and thin film, a balloon having sufficient wearing resistance used for IABP method can be provided.

Balloon of the invention is required to have film thickness of 30 to 80 µm. When the thickness is less than 30 µm and used for IABP method, the balloon tends to be damaged by abrasion caused by calcification at inner side of blood vessels that contact and rub the balloon and burst caused by a pressure impressed inside the balloon. When film thickness of the balloon exceeds 80µm, outer diameter of the folded balloon becomes large. As a result, large perforation for the insert is required and burden to patients increases. Therefore, film thickness of the balloon is preferably 40 to 70 µm, more preferably, 45 to 60 µm. Desired thickness can be obtained such as by selecting the thickness of the parison used for the blow molding.

The balloon of the present invention has 50% modulus in the longitudinal direction of 30 to 140 MPa. When 50% modulus in the longitudinal direction of the balloon is less than 30MPa, wearing resistance becomes insufficient to be used for IABP method. When over 140 MPa, the balloon becomes insufficient to follow bended blood vessels. Balloon of the invention is obtainable by said blow molding with said polyether polyurethane. 50% modulus in the longitudinal direction of the balloon is preferably 50 to 110 MPa, more preferably 60 to 80 MPa.

Further, breaking strength in the longitudinal direction of the balloon is preferably at least 60 MPa. With said breaking strength in the longitudinal direction, the balloon superior in wearing resistance and hard to be damaged by a burst can be obtained. Breaking strength in the longitudinal direction of the balloon is preferably at least 80 MPa, more preferably at least 90 MPa. Upper limit of the breaking strength is not limited but normally at most 190 MPa.

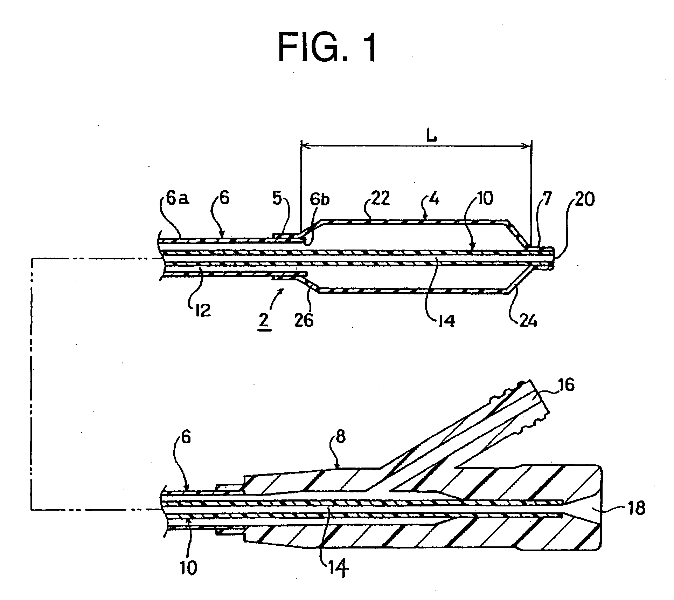

Balloon of the invention, as shown in Fig. 1, is preferably a cylindrical form having cylindrical tube 22 placed at the center of the balloon wherein a distal end side taper 24 forming a thin tip and a distal end 7 are united at distal end of the tube 22, and a proximal end side taper 26 forming a thin tip and a proximal end 5 are united at the proximal end of the tube 22.

Internal capacity of the balloon of the invention is preferably 20 to 50cc, to be suitably used for IABP method. Outer diameter and length of the balloon is determined according to said internal capacity of the balloon, internal diameter of arterial blood vessel, etc. The outer diameter and length L (See Fig. 1) of the balloon when inflated is preferably 10 to 25 mm and 110 to 300 mm respectively.

In the present invention, 50% modulus and breaking strength in the longitudinal direction of the balloon are calculated values obtained by experiments in accordance with JIS K-7311. Here, when measuring said each values, experimental piece was cut in a desired size from molded balloon and drawing experiment was done by drawing the experimental piece in the longitudinal direction of the balloon.

### BRIEF DESCRIPTION OF THE DRAWINGS

Fig. 1 is a sectional view of a IABP catheter according to an embodiment of the present invention;
Fig. 2 is a schematic view of a wearing device used to evaluate wearing resistance property and the use thereof;
Fig. 3 is a schematic sectional view of wearing device as in Fig. 2;
Fig. 4 is a schematic sectional view showing evaluation method of the following ability.

### EMBODIMENTS OF THE INVENTION

Next, an embodiment of IABP catheter fitted with the balloon of the invention is explained below on the basis of figures. As shown in Fig. 1, IABP catheter 2 according to an embodiment of the invention is used for IABP method and comprises balloon 4 , catheter tube 6 and branch part 8.

Balloon 4 is a cylindrical form, which inflate and deflate by a fluid along with heartbeat and is placed at a distal end of balloon catheter 2.

Catheter tube 6 comprises outer tube 6a and inner tube 10, which bore through inside the full length of outer tube 6a along its axial direction. Said catheter tube 6 forms a double-tube structure and balloon 4 is attached and connected to the tube 6 at the distal end.

A first lumen 12 is formed inside the full length of outer tube 6a along its axial direction. The first lumen 12 is placed to let the fluid, which is to inflate the balloon 4, to flow through. Outer surface of distal end 6b of outer tube 6a contacts and connects internal surface of the proximal end 5 of balloon 4. Inner cavity of balloon 4 connects the first lumen 12 formed inside the outer tube 6a.

A second lumen 14 is formed inside the inner tube 10 along its axial direction. A second lumen 14 is placed to lead through a guide wire (not shown), which is to guide balloon 4 to a fixed place inside an artery. Inner tube 10, which bore through the first lumen 12 of outer tube 6a also bore through inner cavity of the balloon 4 . Outer surface of distal end 20 of inner tube 10 contacts and connects internal surface of distal end 7 of balloon 4. Second lumen 14 is opened to outside at the distal end 20. Accordingly, inner cavity of balloon 4 and the first lumen 12 of outer tube 6a will not be connected to the second lumen 14 of inner tube 10. Further, branch part 8 is connected at the proximal end of inner tube 10 and the proximal end of outer tube 6a. Branch part 8 having ports 16 and 18 independently connected to each lumen 12 and 14 respectively.

By the use of IABP catheter 2 manufactured as above, guide wire is lead through the second port 18 of branch part 8 and the second lumen 14 of inner tube 10 and the balloon 4 is lead to a fixed place of an artery. Further, through the first port 16 of branch part 8 and the first lumen 12 of outer tube 6a, a fluid to inflate and deflate the balloon is introduced and derived at inner cavity of balloon 4 . This is to help cardiac function by inflating and deflating the balloon 4 along with heartbeat.

Present invention is not limited to said embodiments and can be varied in various ways within the scope of the invention. For instance, IABP catheter 2 of the invention is not always necessary to have inner tube 10 and can be a single lumen type balloon catheter.

### BEST MODE TO CARRY OUT THE INVENTION

Present invention is concretely described below referring to Examples and Comparative Examples of the invention, however, the invention is not limited to said Examples. Here, 100% modulus, breaking strength and shore A hardness of polyether polyurethane used for molding are calculated values obtained by experimentations in accordance with JIS K-7311 . 50% modulus in a longitudinal direction and breaking strength in a longitudinal direction of the balloon are calculated values from experiments in accordance with JIS K-7311 obtained by cutting experimental pieces from the molded balloon by 100 mm in a longitudinal direction and 10 mm in a circumference direction and drawing the pieces in a longitudinal direction of the balloon.

Further, after the obtained balloon was sufficiently cooled, the balloon was evaluated with its wearing resistance and following ability to follow bended blood vessels by experiments below.

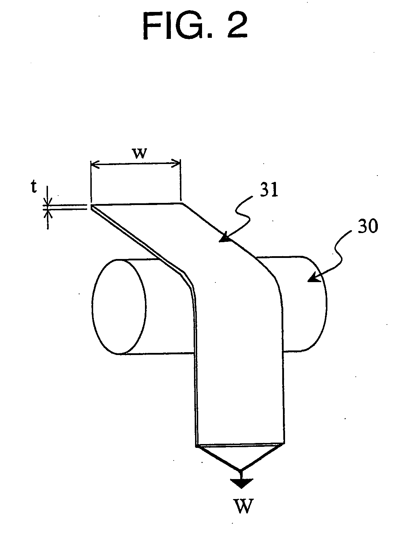

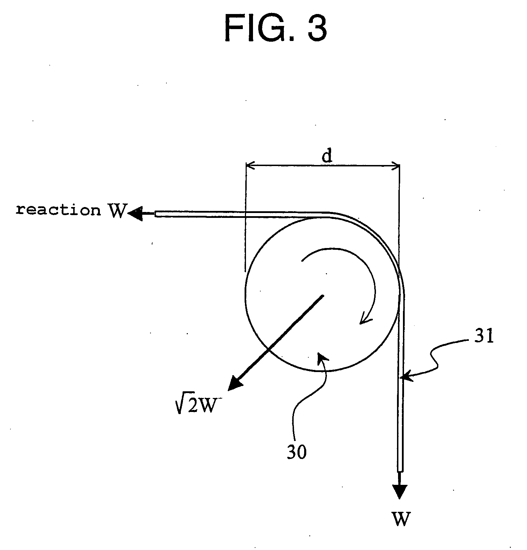

The evaluation of wearing resistance was done by a wearing test using a wearing device as shown in Figs. 2 and 3. Further, plaster roll 30 as shown in Figs. 2 and 3 was formed into a roll shape by shaving massive plaster with a lathe and rubbing its surface by a sandpaper of number 400 for a smooth surface. The roll has a diameter d of 1.6 cm and a length of 10 cm. Experimental piece 31 (a piece obtained by cutting the molded balloon by 100 mm in a longitudinal direction and 5 mm in a circumference direction) was set on the outer surface of the plaster roll 30 as shown in Figs. 2 and 3. And weight was set at an end of the experimental piece 31 and the other end of the same was fixed. Here, load of the weight is W [kg], sectional area of experimental piece 31 is A [cm²] (= width w [cm] of experimental piece 31 x thickness t [cm] of experimental piece 31), and surface area is S [cm²] (=width w x π/4 x diameter d) where plaster roll 30 contacts experimental piece 31. Then, tension T and pressure P on the experimental piece 31 could be obtained by following equations respectively; T = W / A [Pa] and P = √ 2W / S [Pa] . Next, in order to make tension T = 6.86 x 10⁵ Pa and pressure P = 1.96 x 10⁴ Pa, plaster roll 30 was rotated by a rotational speed of 21 cm/sec until experimental piece 31 was cut. Here, film thickness of the obtained balloon was not considered so that tension and pressure of balloons having different film thickness can be evaluated uniformly. Further, said tension and pressure were set approximately the same with the tension and pressure on a balloon at the end term of inflating when IABP catheter is driven.

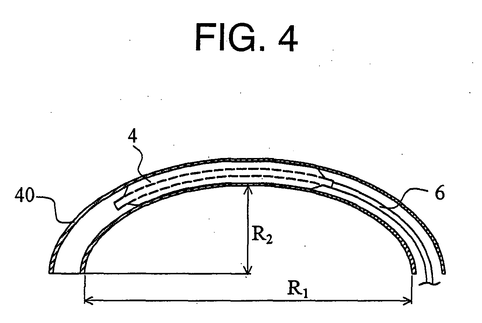

Evaluation on a following ability for bended blood vessels was carried out by a following ability test using device shown in Fig. 4. First, by using obtained balloon, balloon catheter 2 having said structure was manufactured. Next, physiologocal saline solution was poured into a polyvinylchloride tube 40 with the same pressure as in an artery and balloon 4 was inserted into the polyvinylchloride tube 40. The polyvinylchloride tube 40 has inner diameter of 20mm, which is bended to a half elliptical form having a long axis R₁ of 300mm and short axis R₂ of 100mm. Then, when balloon 4 is placed at bended part of polyvinylchloride tube 40, helium gas of approximately 0.018 MPa pressure was sent into balloon 4 through the first lumen 12 of outer tube 6a which lead the balloon 4 to inflate. This inflated balloon 4 was visually observed and when a fold does not occur on the surface and only a small wrinkle occurs, the balloon was considered having sufficient ability to follow bended blood vessels to be used for IABP. Contrary, when a fold was visually observed on balloon 4, it was considered not having sufficient ability to follow bended blood vessels to be used for IABP.

### Example 1

polyether polyurethane (trade name : Pelesen 2363-90AE, Dow Chemical made) having 100% modulus of 10.2 MPa, breaking strength of 41 MPa and shore A hardness of 90 was melt extruded and a parison having outer diameter of 5.2 mm and film thickness of 0.6 mm was manufactured. Next, this parison was set in a balloon mold, drew with magnification of 1.5 in a longitudinal direction while heated at 80 C° , and pressurized inside with a pressure of 0.8 MPa. And the balloon mold was heated to 150 C° and blow molded by making the parison flexible to be expanded. Then, a balloon having a film thickness of 50 µm, a balloon length of 210 mm and an outer diameter of 15 mmwhen inflated was obtained. With the obtainedballoon, 50% modulus in longitudinal direction was 70 MPa, breaking strength in longitudinal direction was 100 MPa, cutting hours of wearing test was 180 minutes. Further, with the following ability test, only many small wrinkles were observed but a fold did not occur.

### Example 2

Balloon having a film thickness of 50 µm, a balloon length of 210 mm and an outer diameter of 15 mm when inflated was obtained in the same way as Example 1, except a material used for manufacturing the balloon. In Example 2, said material for manufacturing the balloon was polyether polyurethane (trade name : Pelesen 2363-80A, Dow Chemical made) having 100% modulus of 6.1 MPa, breaking strength of 36 MPa and shore A hardness of 80. With the obtained balloon, 50% modulus in longitudinal direction was 40 MPa, breaking strength in longitudinal direction was 70 MPa, cutting hours of wearing test was 140 minutes . Further, with the following ability test, only many small wrinkles were observed but a fold did not occur.

### Example 3

Balloon having a film thickness of 50 µm, a balloon length of 210 mm and an outer diameter of 15 mm when inflated was obtained in the same way as Example 1, except a material used for manufacturing the balloon. In Example 3, said material for manufacturing the balloon was polyether polyurethane (trade name : Pelesen 2363-55D, Dow Chemical made) having 100% modulus of 17.2 MPa, breaking strength of 45 MPa and shore A hardness of 97 (shore D hardness of 55). With the obtained balloon, 50% modulus in longitudinal direction was 130 MPa, breaking strength in longitudinal direction was 160 MPa, cutting hours of wearing test was 200 minutes. Further, with the following ability test, only many small wrinkles were observed but a fold did not occur.

### Comparative Example 1

Balloon having a film thickness of 50 µm, a balloon length of 210 mm and an outer diameter of 15 mm when inflated was obtained in the same way as Example 1, except a material used for manufacturing the balloon. In Comparative Example 1, said material for manufacturing the balloon waspolyether polyurethane(trade name:Pelesen 2363-65D, Dow Chemical made) having 100% modulus of 20.0 MPa, breaking strength of 45 MPa and shore A hardness of 98 (shore D hardness of 62) . With the obtained balloon, 50% modulus in longitudinal direction was 180 MPa, breaking strength in longitudinal direction was 200 MPa, cutting hours of wearing test was 220 minutes. With the following ability test, a fold was observed.

### Comparative Example 2

Balloon having a film thickness of 50 µm, a balloon length of 210 mm and an outer diameter of 15 mm when inflated was obtained in the same way as Example 1, except for a material used for manufacturing the balloon. In Comparative Example 2, said material for manufacturing the balloon was polyether polyurethane-(trade name : Pelesen 2103-70A, Dow Chemical made) having 100% modulus of 3.4 MPa, breaking strength of 25 MPa and shore A hardness of 73. With the obtained balloon, 50% modulus in longitudinal direction was 25 MPa, breaking strength in longitudinal direction was 55 MPa, cutting hours of wearing test was 110 minutes. With the following ability test, many small wrinkles were observed but a fold did not occur.

Results of said measurements and evaluations of balloons as in Examples 1 to 3 and Comparative Examples 1-3 are all shown in Table 1.

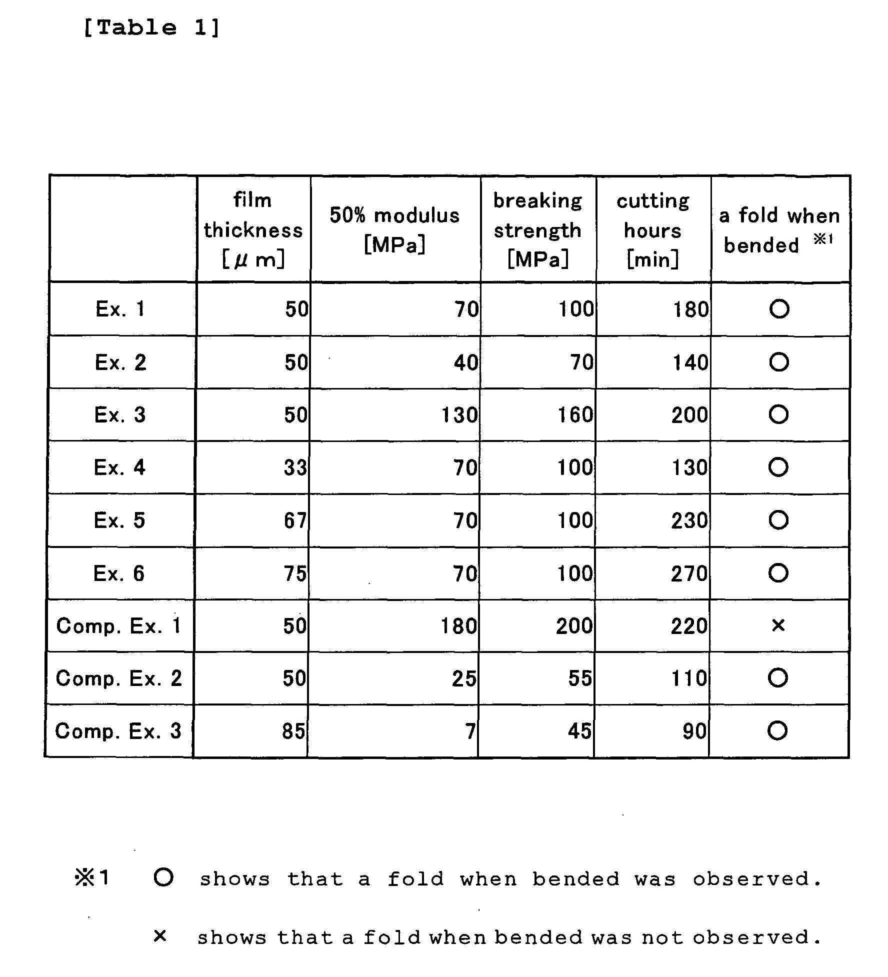

As shown in Table 1, it was confirmed that balloons as in Examples 1 to 3 have extremely long cutting hours compared to the same as in Comparative Examples 2 and 3. Accordingly, as is in Example 1, it can be seen that by blow molding polyether polyurethane having 100% modulus determined in the invention, a balloon having 50% modulus determined in the invention and is superior in wearing resistance can be obtained.

Further, a fold was observed on the balloon as in Comparative Example 1 but was not observed on the same as in Examples 1 to 3. Accordingly, when obtaining a balloon by blow molding, as is in Examples 1 to 3, it can be seen that by blow molding polyether polyurethane having 100% modulus determined in the invention, a balloon having 50% modulus determined in the invention and is superior in following bended blood vessels can be obtained.

### Examples 4 to 6

In Examples 4 to 6, balloons were obtained by blow molding in the same way as Example 1 except thickness of the manufacturing parisons were 0.4, 0.8 and 0.9 mm each and film thickness of the balloons were 33, 67 and 75 µm respectively. The obtained balloons were tested in the same way as Example 1. Results are shown in Table 1. As shown in Example 1, it was confirmed that even film thickness were varied within a range of the invention, i.e. 30 to 80 µm, results equivalent to Examples 1 to 3 can be obtained.

As explained above, according to the invention, by blow molding polyether polyurethane, a balloon and a catheter fitted with the same that are possible to follow bended blood vessels, to carry out low profiling, and to provide sufficient wearing resistance when used for IABP method even with a thin and one layer film can be provided.

## Claims

1. A balloon for Intra Aortic Balloon Pumping catheter manufactured by blow molding polyether polyurethane whose 100% modulus is 5 to 18 MPa, wherein
the balloon has a film thickness of 30 to 80 µm and a 50% modulus in the longitudinal direction of 30 to 140 MPa.

2. The balloon as in claim 1 wherein breaking strength of polyether polyurethane is at least 30 MPa.

3. The balloon as in claim 1 or 2 wherein shore A hardness of polyether polyurethane is 80 to 97.

4. An IABP catheter comprising
a balloon which is able to inflate and deflate along with introduction and derivation of a fluid inside the balloon wherein;
the balloon is produced by blow molding the polyether polyurethane whose 100% modulus is 5 to 18 MPa,
and the balloon has a film thickness of 30 to 80 µm and a 50% modulus in the longitudinal direction of 30 to 140 MPa.

5. A manufacturing method of producing the balloon for IABP catheter as in any one of the claims 1 to 3 comprising the steps of;
manufacturing a parison from polyether polyurethane,
drawing the parison while heating the same,
molding the balloon by inserting the parison in a balloon mold and heating the balloon mold while impressing pressure in the parison and adding tension to the parison in an axial direction, and
removing molded balloon from the mold by cooling the mold to a room temperature while impressing pressure in the parison, then relieving the pressure in the parison.

6. The manufacturing method of producing the balloon as in claim 5 wherein the parison is made from the polyether polyurethane in order to have an outer diameter of 3 to 6 mm and thickness of 0.3 to 2 mm.

7. The manufacturing method of producing the balloon as in claim 5, wherein the parison is heated at 60 to 100 C° and is drew with magnification of 1.0 to 2.0.

8. The manufacturing method of producing the balloon as in claim 5 further comprising the steps of;
inserting the parison into the balloon mold, and
heating the balloon mold to 110 to 190C while impressing pressure in the parison at 0.5 to 1.2MPa and adding tension to the parison in an axial direction .
